(19) Europäisches Patentamt
European Patent Office
Office européen des brevets

(11) Publication number: 0 002 561
B1

(12) EUROPEAN PATENT SPECIFICATION

(45) Date of publication of patent specification: 20.01.82

(51) Int. Cl.³: C 07 D 301/02

(21) Application number: 78300481.5

(22) Date of filing: 10.10.78

(54) **Process for preparing oxirane compounds.**

(30) Priority: 19.12.77 US 861803

(43) Date of publication of application: 27.06.79 Bulletin 79/13

(45) Publication of the grant of the European patent: 20.01.82 Bulletin 82/3

(84) Designated Contracting States: BE DE FR GB LU NL SE

(56) References cited:
DE - A - 2 412 136
DE - A - 2 624 628
DE - A - 2 707 638
DE - A - 2 709 440
US - E - 29 597
US - A - 4 012 423
US - A - 4 012 424

(73) Proprietor: CHEM SYSTEMS, Inc.
747 Third Avenue
New York New York 10017 (US)

(72) Inventor: Sherwin, Martin B
90 Ashburn Road Wayne
Passaic New Jersey 07470 (US)
Inventor: Frank, Marshall E
44 Old Lyme Road Chappaqua
Westchester New York 10514 (US)

(74) Representative: Harrison, Michael Robert et al,
Urquhart-Dykes & Lord Tower House Merrion Way
Leeds, LS2 8PB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 002 561 B1

Courier Press, Leamington Spa, England.

Process for preparing oxirane compounds

A process for the vapor phase production of oxirane compounds is taught in U.S. Patent No. 4,012,423. Although that patent gives a complete description of the process, so much of that disclosure as is necessary for clarity will be repeated in this application. The process utilizes as a starting material a vicinal hydroxyester described by the general formula.

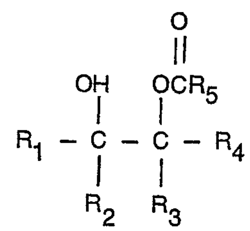

where $R_1$, $R_2$, $R_3$, $R_4$, and $R_5$ may be H, alkyl, alkenyl or alkynyl of about 1 to 16 carbon atoms; an aryl, such as phenyl or naphthyl; cyano;

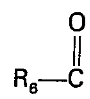

wherein $R_6$ is H, alkyl, alkoxy, carbomethoxy, or carboacyl. Preferably at least two of the aforesaid group are H and the remaining R groups are H, methyl, ethyl, propy, butyl or phenyl. The oxirane compound is prepared by the deacyloxylation of the vicinal hydroxyester in the presence of a basic material. The reaction products are oxirane compounds of the general formula

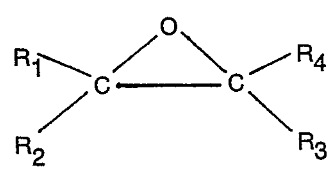

and $R_5COOH$

wherein $R_1$, $R_2$, $R_3$, $R_4$, and $R_5$ are as previously described.

The aforementioned U.S. Patent No. 4,012,423 teaches methods for preparing the oxirane compounds using a batch process with respect to the basic catalyst material. Since it has been found that this catalyst must be periodically regenerated to remove coke and has a useful life on the order of a few months, it is obvious to those skilled in the art that it would be preferred to have a process which provides for a continuous replacement and regeneration of the catalyst material. Conventionally, processes utilizing the continuous replacement of catalysts utilize as the reaction vessel a fluidized bed wherein the catalyst is fluidized and continuously withdrawn and replaced. However, since the reaction of this invention is endothermic, such a system would require heat addition to the reactor as well as additional heating in the catalyst regeneration step.

Catalytic cracking systems have been described wherein the fluid bed catalyst cracker is replaced by a dilute phase catalytic cracker; see for example *Fluidization and Fluid Particle System*, p. 48 et seq., Zens and Othmer, Reinhold, 1960.

It has surprisingly been found that oxirane compounds may be prepared in a continuous process by vapor phase deacyloxylation of vicinal hydroxyesters utilizing a dilute phase transport reactor as the reaction zone and a fluidized bed for heating and reactivating the catalyst. The catalyst is heated in a fluidized bed to the temperature necessary for carrying out the deacyloxylation process and transferred to a dilute phase transport reactor with a co-current stream of reactor feed gas preferably comprising the vicinal hydroxyesters, a solvent for the esters and inert gas. The product gas exits from the transport reactor and is separated from the catalyst in a cyclone separator. The catalyst is returned to the fluidized bed heater and the product gas treated further to recover product.

This invention relates to a continuous process for the preparation of oxirane compounds from a vicinal hydroxyester by vapor phase deacyloxylation of the esters in the presence of a basic material. The overall reaction is described by the equation

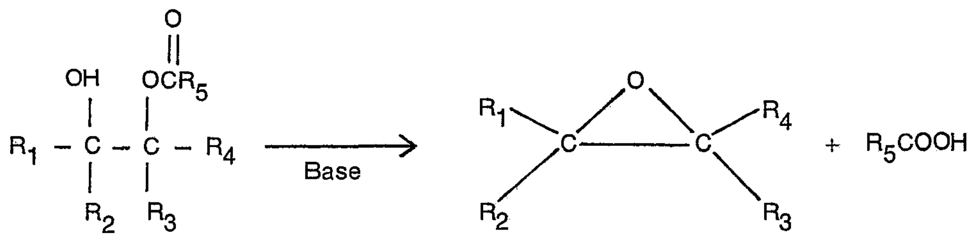


wherein $R_1$, $R_2$, $R_3$, $R_4$, and $R_5$ are as previously described. In the preferred embodiment the vicinal hydroxyester is derived from an olefin compound selected from ethylene, propylene, butylene, pentene, styrene or alpha methyl styrene, and $R_5$ is methyl, ethyl or propyl.

The hydroxyesters may be fed directly to the reaction zone or, if desired, delivered with a carrier gas. The carrier gas acts as a heat sink and also serves to lower the partial pressure of the hydroxyesters in the reaction. The carrier gas may be a liquid, i.e., condensable at room temperatures, such as benzene, toluene, xylene, pr pseudocumene. Non-condensable carrier gases may also be used; these include nitrogen, helium, methane and carbon dioxide. Preferably, the carrier gas comprises the corresponding glycols or glycol diesters of the vicinal hydroxyesters used as feed stock; see, for example, U.S. Patent No. 4,012,424. Generally, where a carrier is used, the hydroxyesters are from 10 to 75 percent by weight, preferably 25 to 60 percent by weight of the feed.

Reaction conditions must be sufficient to maintain the hydoxyesters in the vapor phase. Suitable temperatures for carrying out the reaction will vary depending on the particular hydroxyester, the concentration of the hydroxyester in the carrier gas and the total system pressure.

Generally deacyloxylation of the hydroxyester proceeds at a temperature of 250°C. to 600°C.; preferably 250° to 500°C., and most preferably 350°C. to 450°C. A wide range of pressures may be used, including high pressures up to $2.8 \times 10^6$ Pa (400 psi) and vacuum down to $6.9 \times 10^2$ Pa (0.1 psia). Of paramount importance is the partial pressure of the hydroxyester in the feed stream. Generally, partial pressures over $6.9 \times 10^5$ Pa (100 psi) are not used and for ease of operation atmospheric pressure is often preferred. However, surprisingly it has been found that low pressure operation results in high conversions without loss of selectivity. More specifically, therefore, it is preferred that the partial pressure of the hydroxyester in the feed be $3.45 \times 10^3$ to $1.03 \times 10^5$ Pa (0.5 to 15 psia), more preferably $6.9 \times 10^3$ to $5.5 \times 10^4$ Pa (1 to 8 psia).

The contact time of the hydroxyester within the reactor is 0.001 to 20 seconds, preferably 0.2 to 5 seconds and most preferably 0.5 to 2 seconds.

The catalyst used in this invention is a basic material used *per se* or deposited onto or incorporated into a suitable inert support carrier such as alumina, carborundum or silica gel. The term "catalyst" as used in the specification and claims means both the neat catalyst compound and the catalyst compound on or incorporated into a suitable inert support material.

The basic materials which may be used as catalysts include salts of organic acids and alkali or alkaline earth metals as well as the inorganic salts and oxides of those metals. The preferred catalysts are alkaline silicates and carboxylates. Preferably, the carboxylate is of the same $R_5$ moiety as previously described which makes up the ester group of the vicinal hydroxyester. The preferred catalysts are the silicates and carboxylates of sodium, potassium, lithium, calcium and barium; more preferably, sodium or potassium silicate.

Other compounds which may be used as catalysts are the borates, phosphates, oxides, and carbonates of Group I, II and IIIA metals; see for example U.S. Patent No. 4,012,423.

Non-limiting illustrative examples of catalysts of this invention include sodium acetate, potassium acetate, calcium acetate, sodium borate, potassium metaborate, calcium metaborate, sodium aluminate, sodium silicate and potassium silicate.

Surprisingly, it is preferred that the catalyst not have a high surface area. Preferably the surface area of the catalysts should be 0.15 to 20 square metres per gram; more preferably 0.15 to 10 $M^2$/gm; most preferably 0.15 to 2 $M^2$/gm. At higher surface area to weight ratios there is less selectively toward the formation of the desired products.

As is described in U.S. Patent No. 4,012,423, not wishing to be bound by theory, it is believed that the basic material is not truly catalytic in that it takes part in the reaction. For example, where propylene oxide is to be formed from propylene hydroxyacetate using sodium acetate as the basic material, the acetate portion of the basic material first reacts with the hydroxyl group of the hydroxyacetate, thereby activating the remaining oxygen atom. The activated oxygen attaches to the acetate carbon forming the propylene oxide and splitting off a mole of acetate ion. The acetate ion thus generated reacts with the sodium ion from the original sodium acetate and provides additional sodium acetate which in turn reacts with another mole of the feed. Thus it can be seen that the basic material in the reaction zone is in dynamic mobile equilibrium and is continuously regenerated. This dynamic

equilibrium enables the process to be carried out with a small amount of the basic material, thus giving the basic material the appearance of functioning catalytically.

It is believed that the basic salts and oxides useful as catalysts are converted *in situ* to the carboxylate in the manner described above. Notwithstanding the fact that the theory suggests that the basic material is not a catalyst in the classical sense, the term "catalyst" is used in the specification and claims to describe the basic material and its apparent function in the reaction process.

In the process of this invention the reaction is carried out in a dilute phase transport reactor. The reactor feed gas and the fluidized catalyst are fed into the reactor simultaneously at temperatures sufficient to insure that the temperature in the reactor is at the deacyloxylation temperature. Generally, both gases are in the deacyloxylation temperature range. Preferably, the catalyst is at a higher temperature. The reaction takes place as the catalyst is transported through the reactor. The product effluent is separated from catalyst in a cyclone separator, the catalyst being returned to fluid bed catalyst heater. Secondary cyclone treatment is used to further purify the product stream, which is ultimately treated to recover product.

The catalyst is preferably of a particle size of twenty to two hundred microns. The gas velocity in the transport reactor is six to twenty-one metres per second (twenty to seventy feet per second) while the volume fraction of catalyst in the cracker is 0.25 to 5.0 volume percent.

The term "Dilute Phase Transport Reactor" as used in the specification and claims means the reactor of this invention into which is introduced a catalyst stream and a feed stream while the term "Dilute Phase Transport Reactor System" as used in the specification and claims means the reactor, the fluid bed catalyst heater and the associated equipment used with those units.

Referring now to the figure, the fluid bed catalyst heater, *1*, contains a sufficient inventory of catalyst, *13*, to permit recycling of catalyst, *13*, as herein described. The actual quantity of catalyst, *13*, will depend on the size of the dilute phase transport reactor, *2*, selected and the volume of the reactor feed gas. Since the elements of a fluid bed are substantially standard, the heater, *1*, is shown in schematic. The catalyst, *13*, is heated to approximately 420°C. by a source of salt in heater tubes, *11*. The inert fluidizing medium is introduced at an inlet, *9*, near the bottom of the heater, *1*, below the heater distributor plate. Additionally, steam, *8*, can be introduced with the inert fluidizing gas in an amount of about 20 mole percent based upon the fluidizing gas. Steam serves to activate the catalyst and reduce coking. Alternatively, the catalyst can be regenerated by injection of oxygen into the fluid bed heater or into a diverted stream of catalyst which is reintroduced into the heater.

The catalyst, *13*, is transferred through an outlet line, *7*, to the reactor. A flow of inert stripping gas is introduced countercurrent to the outward flow of catalyst in order to strip out of the catalyst stream any stream which may be carried along with the catalyst. An inert gas is utilized to fluidize the catalyst in the pipe leading to reactor, *2*. The feed gas is introduced through an inlet line, *14*, and intimately mixes with the catalyst, *13*. The reaction is carried out as the catalyst is transported through the reactor, *2*.

The effluent from the reactor, *2*, is fed into a cyclone separator, *3*, to separate the product gas stream from the catalyst, which is returned to the heater, *1*, after being stripped of product gases in a stripping column, *4*. The overhead from the first cyclone is fed through a transfer line, *10*, into a second cyclone, *5*, in order to further separate any catalyst fines which may be carried over from the first cyclone, *3*. The catalyst collected is thereafter transferred to the heater, *11*, through the transfer line, *6*.

The effluent from the second cyclone, *5*, is carried out through a transfer line, *12*, to be further processed to recover product. The inert fluidizing gas used in the heater, *1*, is recycled after clean-up and compression.

Though the sizing of equipment will of course depend on the amount of reactor feed gas to be processed, Table I sets forth typical sizing and flow rates for a process utilizing as the catalyst potassium silicate of approximately 50 microns in diameter and as the feed gas the hydroxyacetate ester of propylene. Although the figure shows a single reactor used in conjunction with the fluid bed catalyst heater, *1*, the design is such that using the equipment size and parameters which are presented in Table I and the Figure, four such reactors would be required with the catalyst heater described.

TABLE I

*Fluid Bed Catalyst Heater*
Dimensions — Diameter — 6.1 m (20 ft). Height 12.2 m (40 ft).
Fluidizing Gas Flow — 6728 $m^3$ per hr (237,600 Cubic ft. per hr).
Steam Flow — 454 Kg/hr (1,000 lbs/hr).
Catalyst Inventory — 6.8 × $10^4$ Kg (150,000 lbs).
Pressure Drop across Fluidized Bed — 5.24 × $10^4$ Pa (*ca.* 7.6 psia).
Circulating Salt Heater Capacity — 1.00 × $10^{11J}$ (95.1 × $10^6$ BTU/hr).

TABLE I (continued)

*Reactor Dimensions:*
Diameter — 1.94 m (6.36 ft). Height — 14.6 m (48 ft).
Catalyst Flow to the Reactors — 4.4 x $10^6$ Kg/hr (9.6 x $10^6$ lbs/hr).
Reactor Feed Gas Temperature — 375°C.
Total Reactor Feed Gas — 6.76 x $10^5$ Kg/hr (1.49 x $10^6$ lbs/hr)
Reactor Feed Gas Composition — propylene glycol monoacetate 31.3 wt. %; propylene glycol diacetate 68.0 wt. %; propylene glycol 0.7%.

It will be obvious to those skilled in the art that the size of equipment and flow rate of feed gas is not a critical limitation of the process. The aforegoing example of Table I is merely by way of illustration.

Although a conventional fluidized bed reactor could be used to prepare an oxirane compound, such a method has certain disadvantages. There is considerable backmixing and long contact times. Backmixing causes reverse reactions and reduces the driving force of the reaction with a consequent lower conversion and selectivity. Additionally, if steam is introduced into the fluid bed reactor to reduce coking, the steam results in water in the condensed product stream which causes hydrolysis of the product during separation. A comparison of various prior art catalytic cracking methods with the method of this invention is set forth in Table II below.

TABLE II

| Reactor | Fixed Bed | Conventional Fluidized Bed | Dilute Phase[2] Transport |
|---|---|---|---|
| Catalyst size | 3.2 x $10^{-3}$ m (1/8 inch) | 30—80 micron | 30—80 micron |
| Gas velocity (ft./sec.) | 1.5 x $10^{-1}$ m/s (0.5) | 4.6 x $10^{-2}$ m/s (0.15) | 15.3 m/s 50 |
| Empty tube contact time (sec.) | 1.0 | 3.0 | 0.8 |
| PGMA[1] conversion (%) | 35 | 60 | 40 |
| PGMA selectivity to propylene oxide (%) | 83 | 55 | 86 |

1 PGMA = propylene glycol monoacetate
2 Calculations based on system kinetics and gas velocity effects.

It can be seen from Table II that in addition to avoiding problems referred to above with fluidized bed reactors, the selectivity of the present process is substantially greater than that of the fluidized bed reactor process. Furthermore, the percent conversion is higher than that for a fixed bed reactor. In fixed bed reactors it is difficult to maintain uniform temperatures and therefore selectivity problems result. Additionally, the heat supply requirements of such a system are much greater than in the system of the present invention. Furthermore, means for catalyst regeneration and replacement require cumbersome valving and other equipment.

**Claims**

1. A process for preparing an oxirane compound by the deacyloxylation of a vicinal hydroxyester using a catalyst a basic material, characterized by carrying out the process continuously in a dilute phase transport reactor by:

(a) heating a fluidized bed of catalyst in a heating zone to the temperature necessary for carrying out the deacyloxylation process;

(b) introducing said heated catalyst and a preheated feed gas stream into the reactor, wherein the deacyloxylation of a vicinal hydroxyester component of the feed gas is accomplished, thereby producing an oxirane compound;

(c) separating the reacted gas stream from the catalyst;

(d) returning the catalyst to the fluidised bed in the heating zone for reheating; and

(e) further processing the separated reacted gas stream to separate out the oxirane product.

2. The process of claim 1 where the catalyst is supported on an inert support material.

3. The process of claims 1 or 2 characterized in that the catalyst support material comprises alumina, carborundum or silica gel.

4. The process of any one of claims 1 to 3 characterized in that the gas feed stream comprises a vicinal hydroxyester, an organic solvent for said ester, and an inert gas.

5. The process of claim 4 characterized in that the catalyst is fluidized by an inert gas wherein the inert gas is nitrogen, carbon dioxide or methane.

6. The process of any one of claims 1 to 5 characterized in that the total pressure in the reactor is sub-atmospheric.

7. The process of any one of claims 1 to 6 characterized in that the hydroxyester is propylene hydroxyacetate and the catalyst is potassium silicate or sodium silicate.

8. The process of claim 6 characterized in that the partial pressure of the vicinal hydroxyacetate in the reactor is $6.9 \times 10^3$ to $5.5 \times 10^4$ Pa (1 psia to 8 psia).

9. The process of any one of claims 1 to 8 characterized in that the catalyst is potassium silicate or sodium silicate.

10. The process of any one of claims 1 to 9 characterized in that the catalyst surface area is 0.15 to 20 square metres per gram.

11. The process of claim 10 characterized in that the catalyst surface area is 0.15 to 2.0 square metres per gram.

12. The process of any one of claims 1 to 11 characterized in that the catalyst is reactivated in the heating zone by contacting with a reactivating gas.

13. The process of claim 12 characterized in that reactivating gas is steam.

14. The process of claim 12 characterized in that the reactivating gas is oxygen.

15. The process of any one of claims 1 to 14 characterized in that the heated catalyst is at a temperature higher than the feed gas, so that the feed gas is further heated by the catalyst.

**Revendications**

1. Procédé de préparation d'un composé d'oxyrane par la désacyloxylation d'un hydroxyester vicinal et utilisant, comme catalyseur, un matériau basique, caractérisé en ce que le procédé est effectué continuellement dans un réacteur à transport de phase diluée en:

(a) chauffant un lit fluidisé d'un catalyseur dans une zone de chauffage à la température nécessaire pour effectuer le procédé de désacyloxylation;

(b) introduisant ledit catalyseur chauffé et un courant d'un gaz alimentaire pré-chauffé dans le réacteur, où la désacyloxylation d'un composant d'hydroxyester vicinal du gaz alimentaire est accomplie, afin de produire ainsi un composé d'oxyrane;

(c) séparant le courant de gaz ayant réagi du catalyseur;

(d) ramenant le catalyseur au lit fluidisé dans la zone de chauffage pour le réchauffer;

(e) traitant encore le courant de gaz ayant réagi et séparé pour en séparer l'oxyrane produit.

2. Procédé selon la revendication 1, caractérisé en ce que le catalyseur est supporté sur un matériau de support inerte.

3. Procédé selon l'une des revendications 1 ou 2, caractérisé en ce que le matériau de support du catalyseur comprend de l'alumine, du carborundum ou du gel de silice.

4. Procédé selon l'une des revendications 1 à 3, caractérisé en ce que le courant de gaz alimentaire comprend un hydroxyester vicinal, un solvant organique dudit ester et un gaz inerte.

5. Procédé selon la revendication 4, caractérisé en ce que le catalyseur est fluidisé par un gaz inerte, ledit gaz inerte étant de l'azote, du gaz carbonique ou du méthane.

6. Procédé selon l'une des revendications 1 à 5, caractérisé en ce que la pression totale dans le réacteur est sub-atmosphérique.

7. Procédé selon l'une des revendications 1 à 6, caractérisé en ce que l'hydroxyester est de l'hydroxyacétate de propylène et en ce que le catalyseur est du silicate de potassium ou du silicate de sodium.

8. Procédé selon la revendication 6, caractérisé en ce que la pression partielle de l'hydroxyacétate vicinal dans le réacteur est de $6,9 \times 10^3$ à $5.5 \times 10^4$ Pa.

9. Procédé selon l'une des revendications 1 à 8, caractérisé en ce que le catalyseur est du silicate de potassium ou du silicate de sodium.

10. Procédé selon l'une des revendications 1 à 9, caractérisé en ce que l'aire superficielle du catalyseur est de 0,15 à 20 $m^2/g$.

11. Procédé selon la revendication 10, caractérisé en ce que l'aire superficielle du catalyseur est de 0,15 à 2,0 $m^2/g$.

12. Procédé selon l'une des revendications 1 à 11, caractérisé en ce que le catalyseur est réactivé dans la zone de chauffage par contact avec un gaz réactivant.

13. Procédé selon la revendication 12, caractérisé en ce que le gaz réactivant est de la vapeur.

14. Procédé selon la revendication 12, caractérisé en ce que le gaz réactivant est de l'oxygène.

15. Procédé selon l'une des revendications 1 à 14, caractérisé en ce que le catalyseur chauffé est à une température supérieure au gaz alimentaire, ainsi le gaz alimentaire est encore chauffé par le catalyseur.

**Patentansprüche**

1. Verfahren zur Herstellung einer Oxiranverbindung durch Desacyloxylierung eines vizinalen Hydroxyesters unter Verwendung eines basischen Materials als Katalysator, dadurch gekennzeichnet, daß das Verfahren kontinuierlich in einem Reaktor durchgeführt wird, der für den Transport von verdünnter Phase vorgesehen ist, indem man

(a) eine Katalysatorwirbelschicht in einer Heizzone auf eine Temperatur erhitzt, die zur Durchführung des Desacyloxylierungsverfahrens erforderlich ist,

(b) den erhitzten Katalysator und einem vorerhitzten Zufuhrgasstrom in den Reaktor einführt, in dem die Desacyloxylierung einer vizinalen Hydroxyesterkomponente des Zufuhrgases unter Bildung einer Oxiranverbindung vollendet wird,

(c) den umgesetzten Gasstrom von dem Katalysator abtrennt,

(d) den Katalysator zu der Wirbelschicht in der Heizzone zum Wiederaufheizen zurückführt und

(e) den abgetrennten, umgesetzten Gasstrom weiterverarbeitet, um das Oxiranprodukt abzutrennen.

2. Verfahren nach Anspruch 1, dadurch gekennzeichnet, daß der Katalysator auf einem inerten Trägermaterial aufgebracht wird.

3. Verfahren nach Anspruch 1 oder 2, dadurch gekennzeichnet, daß das Katalysatorträgermaterial Aluminiumoxid, Siliciumcarbid oder Silikagel ist.

4. Verfahren nach einem der Ansprüche 1 bis 3, dadurch gekennzeichnet, daß der Gaszufuhrstrom einen vizinalen Hydroxyester, ein organisches Lösungsmittel für den Ester und ein Inertgas enthält.

5. Verfahren nach Anspruch 4, dadurch gekennzeichnet, daß der Katalysator durch ein Inertgas aufgewirbelt wird, wobei als Inertgas Stickstoff, Kohlendioxid oder Methan verwendet wird.

6. Verfahren nach einem der Ansprüche 1 bis 5, dadurch gekennzeichnet, daß der Gesamtdruck in dem Reaktor unter Atmosphärendruck liegt.

7. Verfahren nach einem der Ansprüche 1 bis 6, dadurch gekennzeichnet, daß der Hydroxyester Propylenhydroxyacetat und der Katalysator Kaliumsilicat oder Natriumsilicat ist.

8. Verfahren nach Anspruch 6, dadurch gekennzeichnet, daß der Partialdruck des vizinalen Hydroxyacetats im Reaktor $6{,}9\times10^3$ bis $5{,}5\times10^4$ Pa beträgt.

9. Verfahren nach einem der Ansprüche 1 bis 8, dadurch gekennzeichnet, daß der Katalysator Kaliumsilicat oder Natriumsilicat ist.

10. Verfahren nach einem der Ansprüche 1 bis 9, dadurch gekennzeichnet, daß die Katalysatoroberfläche 0,15 bis 20 $m^2/g$ beträgt.

11. Verfahren nach Anspruch 10, dadurch gekennzeichnet, daß die Katalysatoroberfläche 0,15 bis 2,0 $m^2/g$ beträgt.

12. Verfahren nach einem der Ansprüche 1 bis 11, dadurch gekennzeichnet, daß der Katalysator in der Heizzone reaktiviert wird, indem er mit einem reaktivierenden Gas in Berührung gebracht wird.

13. Verfahren nach Anspruch 12, dadurch gekennzeichnet, daß das reaktivierende Gas Dampf ist.

14. Verfahren nach Anspruch 12, dadurch gekennzeichnet, daß das reaktivierende Gas Sauerstoff ist.

15. Verfahren nach einem der Ansprüche 1 bis 14, dadurch gekennzeichnet, daß der erhitzte Katalysator bei einer höheren Temperatur als das Zufuhrgas gehalten wird, so daß das Zufuhrgas durch den Katalysator weiter aufgeheizt wird.

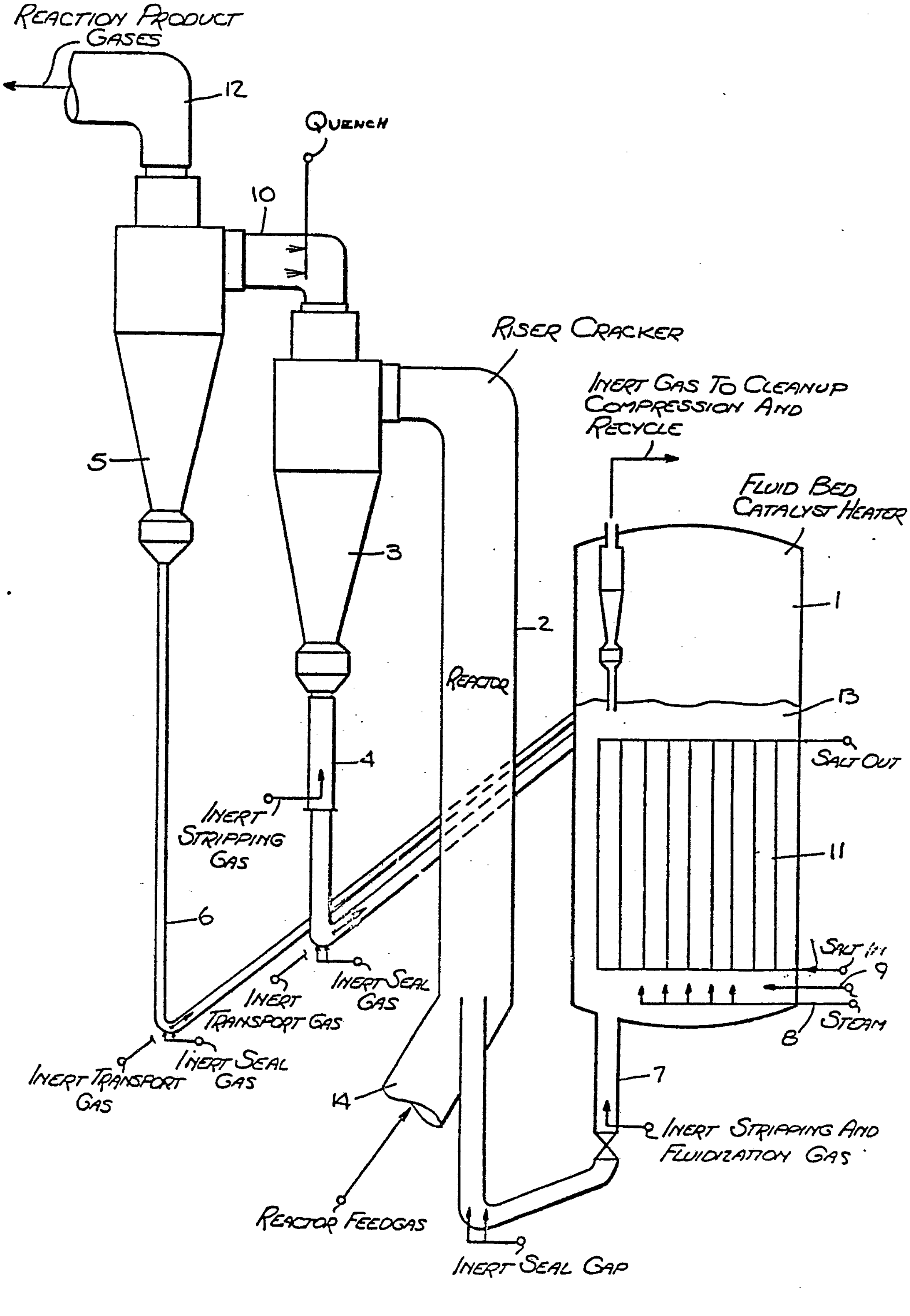
REACTION PRODUCT GASES
12
QUENCH
10
RISER CRACKER
INERT GAS TO CLEANUP COMPRESSION AND RECYCLE
FLUID BED CATALYST HEATER
5
3
1
2
REACTOR
13
SALT OUT
4
INERT STRIPPING GAS
11
6
SALT IN
9
INERT SEAL GAS
INERT TRANSPORT GAS
STEAM
8
INERT TRANSPORT GAS
INERT SEAL GAS
7
14
INERT STRIPPING AND FLUIDIZATION GAS
REACTOR FEEDGAS
INERT SEAL GAP